# EUROPEAN PATENT SPECIFICATION

(11) **EP 4 260 704 B1**
(45) Date of publication and mention of the grant of the patent: **11.03.2026**
(21) Application number: 23167181.9
(22) Date of filing: 06.04.2023
(51) Int. Cl.: A61K 31/17, A61K 31/235, A61K 31/36, A61K 31/4427, A61K 45/06, A61P 31/04, A01N 37/06, A01N 37/30, A01P 1/00

(54) **ANTIMICROBIAL COMPOSITIONS COMPRISING A PHENYL UREA COMPOUND AND A CIS-UNSATURATED FATTY ACID WITH 16 TO 22 CARBON ATOMS**
ANTIMIKROBIELLE ZUSAMMENSETZUNGEN, BESTEHEND AUS EINER PHENYLHARNSTOFFVERBINDUNG UND EINER CIS-UNGESÄTTIGTEN FETTSÄURE MIT 16 BIS 22 KOHLENSTOFFATOMEN
COMPOSITIONS ANTIMICROBIENNES COMPRENANT UN COMPOSE DE PHENYLUREE ET UN ACIDE GRAS CIS-INSATURE DE 16 A 22 ATOMES DE CARBONE

(30) Priority: 13.04.2022 US 202263330325 P
(43) Date of publication of application: 18.10.2023
(73) Proprietor: National Taiwan University, Taipei 10617 (TW); National Yang Ming Chiao Tung University, Taipei City 112304 (TW)
(72) Inventor: CHIU, Hao-Chieh, Taipei (TW); SHIAU, Chung-Wai, 112304 Taipei City (TW)
(74) Representative: 2K Patentanwälte Blasberg Kewitz & Reichel

(56) References cited:
- WO-A1-2011/019834
- WO-A1-2016/115639
- WO-A2-2013/155047
- WO-A2-2017/207556

## Description

### TECHNICAL FIELD

The present invention relates to an antimicrobial composition and related methods; in particular, to an antimicrobial composition, a pharmaceutical composition comprising the same and a use thereof. However, the present invention is not limited thereto.

### BACKGROUND

Pathogens that can cause diseases in humans exist in a variety of forms and have a huge impact on global public health. The most common pathogens are pathogenic bacteria and fungi. *Staphylococcus aureus,* in particular, is currently one of the most common infectious pathogens in developed countries, causing a wide range of diseases from skin infection to deadly pneumonia and septicemia. Moreover, *Staphylococcus aureus* can form persister cells and biofilms that are antibiotic-resistant and hence conducive to chronic and recurring infections. In fact, *Staphylococcus aureus* has developed resistance to most antibiotics, making it more and more difficult and complicated to treat diseases associated with *Staphylococcus aureus.*

In light of the above, the prior art has not a few antibacterial means against *Staphylococcus aureus* strains that are multidrug-resistant. For example, the inventor of the present invention has found that a derivative of Sorafenib, which is a tyrosine kinase inhibitor originally used as an anticancer drug, can depolarize and penetrate bacterial biofilms and thereby exhibit effective antibacterial activity against a plurality of bacterial strains (see Taiwan Invention Patent No. I675817B). In addition, a research has shown that PK150, a Sorafenib analog, can be used in submicromolar concentrations against various pathogenic bacterial strains and has oral bioavailability and *in vivo* efficacy (Le, P et al. Nat. Chem. 12, 145-158 (2020)). Document WO2011019834A1 discloses fatty acid-derived biomaterials, methods of making the biomaterials, and methods of loading them with silver compounds. The silver-containing biomaterials can be utilized alone or in combination with a medical device for the release and local delivery of one or more anti-infective agents. Methods of forming and tailoring the properties of said biomaterials and methods of using said biomaterials for treating injury in a mammal. Document WO2013155047A2 discloses screening assay for identifying inhibitors of Pseudomonas aeruginosa CFTR Inhibitory Factor as well as compounds identified by the screening assay for use in compositions and methods for ameliorating or treating a respiratory disease such as cystic fibrosis or secondary infection thereof. Document WO2017207556A2 discloses compounds which are suitable for treating bacterial diseases and to pharmaceutical compositions containing such compounds. The invention further relates to a kit of parts comprising such compounds and to the use of such compounds as disinfectants. Document WO2016115639A1 discloses antibacterial compositions comprising at least one unsaturated fatty acid or a pharmaceutically acceptable salt thereof; at least one alpha-hydroxy acid or a pharmaceutically acceptable salt thereof; and at least one amino alcohol. The compositions have broad-spectrum antibacterial activity, including on pathogens displaying multi-drug resistance.

### SUMMARY OF THE INVENTION

Based on the prior art, the inventor of the present invention has found during a development process that certain amide compounds and certain fatty acids can work together to produce a synergistic bactericidal effect. Using such a fatty acid in conjunction with such an amide compound not only allows the amide compound to be used in a relatively small amount to produce a therapeutic effect against a drug-resistant bacterial strain, but also can eliminate the persister cells and biofilms of such a microorganism effectively and rapidly.

Accordingly, the present invention provides an antimicrobial composition for use in the treatment of bacterial infection according to claim 1. Advantageous embodiments are the subject of the dependent claims. The antimicrobial composition comprises an amide compound and a fatty acid; wherein the fatty acid is a cis-unsaturated fatty acid with 16 to 22 carbon atoms.

In a first alternative, the amide compound is a compound having chemical structure (I-1) or (I-2): 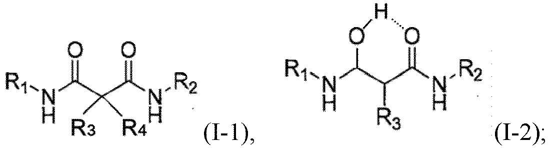 wherein each of R₁ and R₂ is 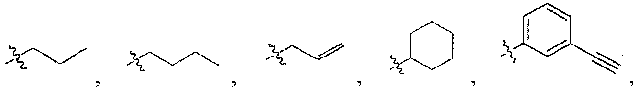 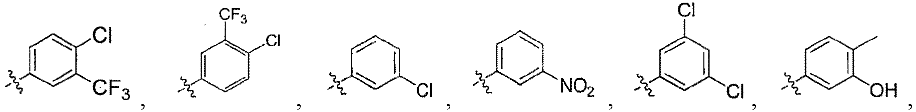 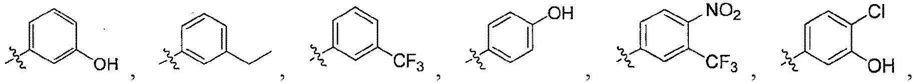 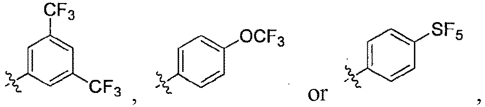 and R₁ and R₂ are not concurrently 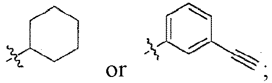 R₃ is hydrogen, toluene, 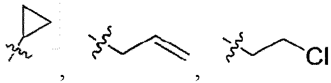 or 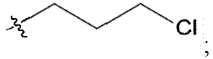 and R₄ is hydrogen or fluorine.

In a second alternative, the amide compound is a compound having chemical structure (II): 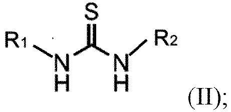 wherein R₁ is 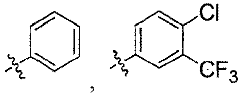 or 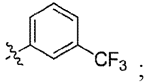 and R₂ is

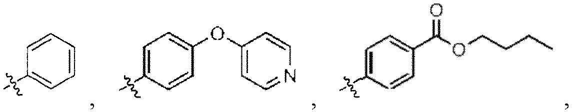

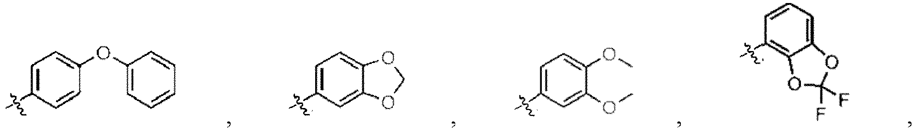

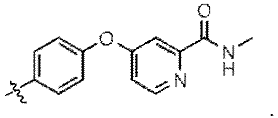

In one embodiment, the amide compound is according to the first alternative and R₁ is 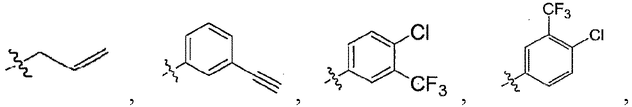 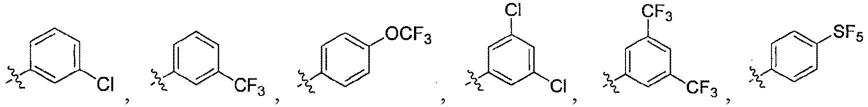 or

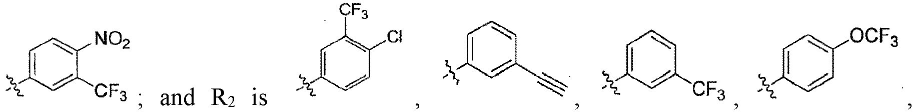

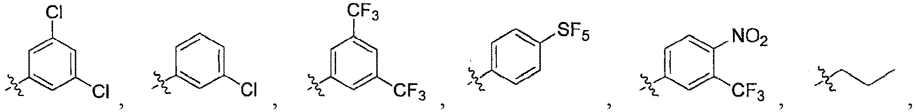

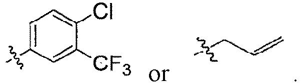

In one embodiment, the amide compound is a compound having any of chemical structure (III) to (VII):

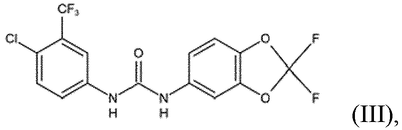

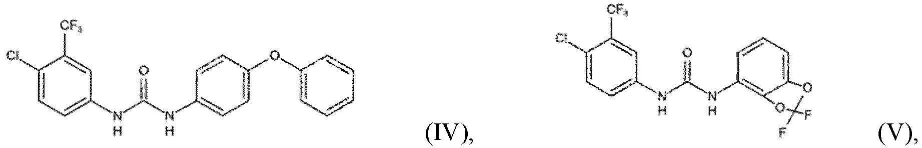

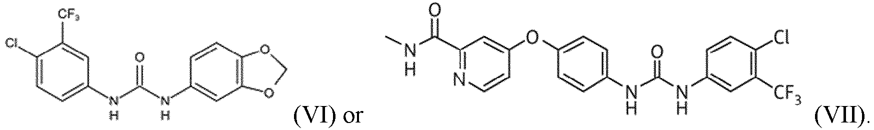

In one embodiment, the fatty acid is a cis-monounsaturated fatty acid or a cis-polyunsaturated fatty acid with 16 to 22 carbon atoms.

In one embodiment, the fatty acid is palmitoleic acid, oleic acid, linoleic acid, α-linolenic acid, arachidonic acid, eicosapentaenoic acid, or docosahexaenoic acid.

In one embodiment, the content of the fatty acid is greater than the content of the amide compound.

In another aspect, the present invention provides a pharmaceutical composition, comprising the antimicrobial composition and at least one pharmaceutically acceptable carrier.

In one embodiment, the microbial cell is a cell of a human pathogenic bacterium.

In one embodiment, the human pathogenic bacterium is selected from a group consisting of *Staphylococcus aureus* (*S. aureus*), *Staphylococcus haemolyticus* (*S. haemolyticus*)*, Staphylococcus hominis* (*S. hominis*)*, Staphylococcus intermedius* (*S. intermedius*)*, Staphylococcus saprophyticus* (*S. saprophyticus*)*, Staphylococcus lugdunesis* (*S. lugdunesis*)*, Erysipelothrix rhusiopathiae, Enterococcus faecalis*, *Enterococcus faecium, VR-E. faecium, Bacillus cereus, Bacillus subtilis, Corynebacterium diphtheriae, Listeria monocytogenes, Streptococcus pyogenes, Clostridium difficile, Escherichia coli, Salmonelia Typhimurium, Acinetobacter baumannii, Mycobacterium tuberculosis,* methicillin-resistant *Staphylococcus aureus* (MRSA) and vancomycin-resistant *Staphylococcus aureus* (VRSA).

In one embodiment, the microbial biofilm is a biofilm of a human pathogenic bacterium or fungus.

In one embodiment, the human pathogenic bacterium or fungus is selected from a group consisting of *Staphylococcus haemolyticus, Klebsiella pneumoniae, Pseudomonas aeruginosa, Escherichia coli, Enterococcus faecalis*, *Enterococcus faecium, Candida albicans* and *Staphylococcus aureus.*

The antimicrobial compositions provided by the present invention use the synergy of an amide compound and a fatty acid to not only allow the amide compound to be used in a relatively small amount to produce a therapeutic effect against a drug-resistant bacterial strain, but also eliminate the persister cells and biofilms of such a microorganism effectively and rapidly.

### BRIEF DESCRIPTION OF THE SEVERAL VIEWS OF THE DRAWINGS

The above and other objectives, features, and advantages of the present invention can be better understood by referring to the following detailed description of some illustrative embodiments in conjunction with the accompanying drawings, in which:

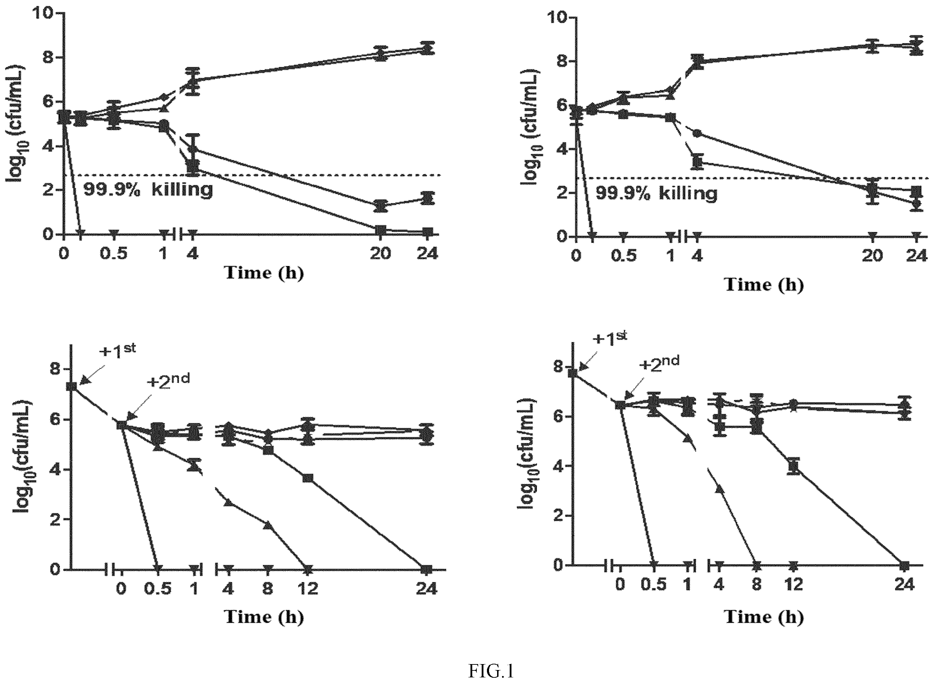

Figure 1: *S. aureus* NCTC8324 (the upper left graph) and MRSA USA300 (the upper right graph) were assayed after exposure to SC5005 (4x MIC, 1 mg/L; filled square), DHA (8 mg/L; filled inverted triangles), SC5005 combined with DHA (filled triangle) and vancomycin (VAN; 4x MIC, 4mg/L; filled circle). The number of viable bacteria in broth after each exposure period was enumerated by a cfu assay and expressed as cfu/mL; the data shown are the mean ± SD (standard deviation) of independent experiments. *S. aureus* NCTC8325 (the lower left graph) and MRSA USA300 (the lower right graph) were first treated with vancomycin (VAN; 16x MIC, 16 mg/L) in PBS for 12 hours to trigger persistence, followed by washing of bacterial cells, and then were assayed after respectively exposure to SC5005 (16x MIC, 4 mg/L; filled squares), DHA (8 mg/L; filled inverted triangles), SC5005 combined with DHA (filled triangles), vancomycin (VAN; 16x MIC, 16 mg/L; filled circle) and daptomycin (DAP; 16x MIC, 16 mg/L; filled star). The number of viable bacteria in the broth after each exposure period was calculated by a cfu assay (cfu/mL); the data are shown as the mean ± SD of three independent experiments (n = 3 for each group). Since the standard deviation of each point is very small, the error line is not shown in each graph. The horizontal dashed line in the upper left graph and the upper right graph represents 99% of the bacterial cells killed. In addition, the blank controls in Figure 1 are shown as filled diamonds.

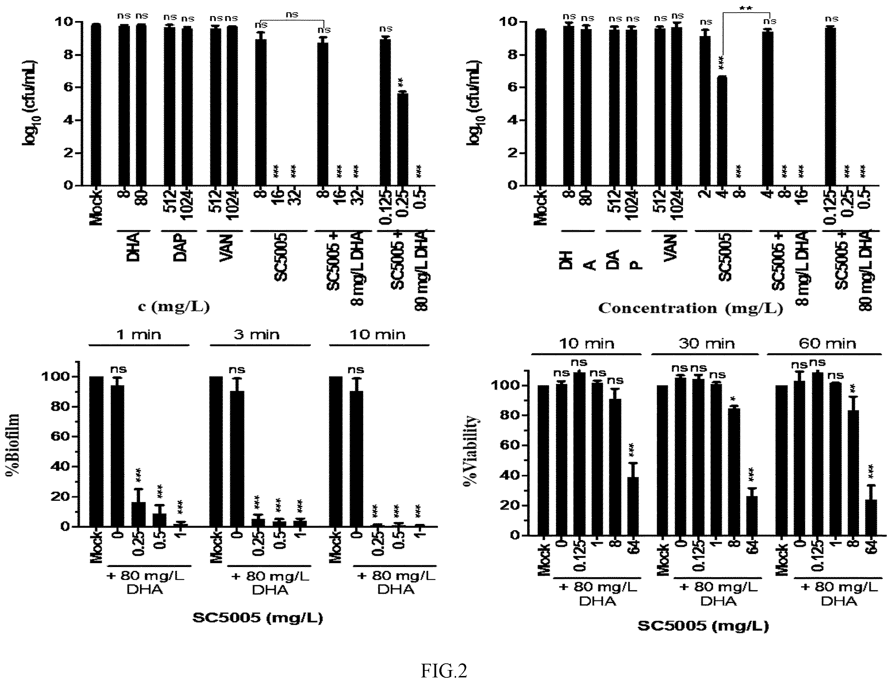

Figure 2: The biofilms of *S. aureus* NCTC8325 (the upper left graph) and MRSA USA300 (the upper right graph) were treated with increasing concentrations of blank control (Mock), DHA, daptomycin (DAP), vancomycin (VAN), SC5005, and SC5005 in combination with DHA for 24 hours. The number of viable bacteria was then determined using a cfu assay. The data represent individual experiments and are shown as the mean ± SD (n = 3 per group). ns, nonsignificant, *p* > 0.05; ** p* < 0.05; *** p <* 0.01; *** *p* < 0.001. (The lower left graph) the biofilms of MRSA USA300 were treated with blank control (Mock) or SC5005 along with 80 mg/L DHA for 1, 3 or 10 minute(s). The viable bacteria in the biofilms harvested and enumerated using a cfu assay. The data represent independent experiments and are shown as mean ± SD (n = 3 per group). ns, nonsignificant, p > 0.05; * p < 0.05; ** p < 0.01; *** p < 0.001 (The lower right graph) RAW264.7 cells were exposed to a blank control (Mock) or increasing concentrations of SC5005 and 80 mg/L DHA for 10, 3or 60 minutes. The viability of cells was determined by the MTT assay and expressed as a percentage relative to mock (DMSO and ethanol)-treated cells. The data shown are the mean ± SD (n = 3 per group).

### DETAILED DESCRIPTION

### Terminology

The terms used in this specification are generally within the scope of the invention and the specific context of each term has the same general meaning as it has in the relevant field. The specific terms used in the description of the invention in this specification will be described below or elsewhere in this specification to assist those skilled in the art in understanding the relevant description of the invention. The same terminology has the same scope and meaning in the same context. In addition, because there is more than one way to express one thing; therefore, terms discussed in this specification may be replaced with alternative terms and synonyms, and there is no particular meaning to be given to whether a term is specified or discussed in this specification. Although this specification provides synonyms for some terms, the use of one or more synonyms does not preclude the use of other synonyms.

As used in this specification, "one" and "the" may also be construed as plural unless the context clearly indicates otherwise. In addition, the numerical ranges and parameters used to define the invention in this specification and the accompanying scope of patent application are approximate values, and the relevant values in specific embodiments have been presented herein as precisely as possible. However, any numerical value will inevitably contain standard deviations due to individual test methods. Here, "approximate" usually means that the actual value is within plus or minus 10%, 5%, 1% or 0.5% of a particular value or range. Alternatively, the term "approximate" means that the actual value falls within the acceptable standard deviation of the mean value, which depends on those with ordinary knowledge in the field of the invention. Therefore, unless otherwise stated to the contrary, the numerical parameters disclosed in this specification and in the scope of the accompanying patent application are approximate values and are subject to change as needed. At a minimum, these numerical parameters should be understood as the indicated effective digits and the values obtained by applying the general rounding method. Furthermore, the description may be accompanied by headings and subheadings for ease of reading, but these headings do not affect the scope of the invention.

As used herein, the term "composition" is intended to include a product containing specified ingredients (each in a specified amount if such amounts are specified) and any product that is directly or indirectly formed by a combination of the specified ingredients in their respective specified amounts.

As used herein, the term "fatty acid" refers to an amphiphilic molecule composed of a hydrophobic carbon chain and a hydrophilic carboxyl group. A fatty acid in which the carbon chain lacks a double bond is referred to as a saturated fatty acid, and a fatty acid whose carbon chain has a double bond is referred to as an unsaturated fatty acid. Unsaturated fatty acids can be further divided by the number of double bonds into monounsaturated fatty acids and polyunsaturated fatty acids. The aforesaid double bond may be a cis-double bond or a trans-double bond, the two of which lead to different physical properties respectively. The majority of unsaturated fatty acids in nature are cis-fatty acids. Trans-fatty acids are mostly generated by hydrogenation.

As used herein, the term "amide compound" refers to a compound of the general formula R₁C(=O)NR₂R₃, where R₁, R₂, and R₃ are substituents and may each be a hydrogen atom or organic group.

As used herein, the term "pharmaceutical composition" refers to a composition that includes an active ingredient and a pharmaceutically acceptable carrier, and the active ingredient referred to herein is an antimicrobial composition provided by the present invention. A pharmaceutical composition may further include a pharmaceutically acceptable excipient or diluent as needed.

As used herein, the term "pharmaceutically acceptable" means that the diluent, excipient, or carrier it modifies is compatible with the other ingredients in the formulation and is harmless to its recipient.

The term "pharmaceutically acceptable carrier" is recognized in the art and refers to a pharmaceutically acceptable material, composition, or excipient that is suitable for administering an antimicrobial composition of the invention to a mammal.

### Antimicrobial composition

The present invention provides an antimicrobial composition comprising: an amide compound; and a fatty acid; wherein the fatty acid is a cis-unsaturated fatty acid with 16 to 22 carbon atoms.

According to some embodiments of the present invention, the amide compound is a malonamide derivative; preferably, it is a compound having the chemical structure (I-1) or (I-2): 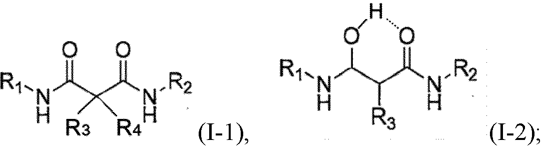 wherein each of R₁ and R₂ is 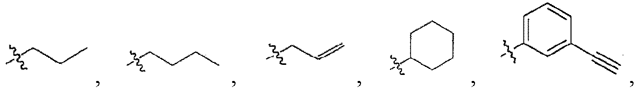 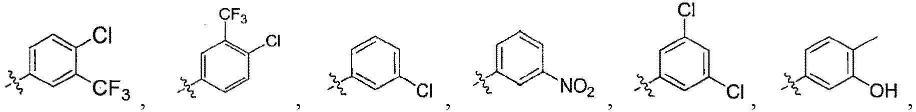 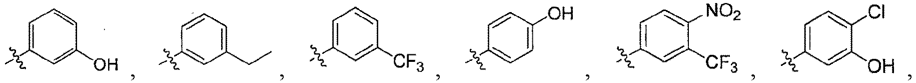 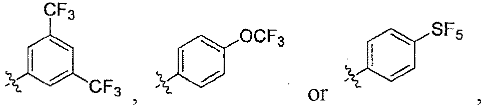 and R₁ and R₂ are not concurrently 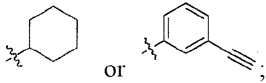 R₃ is hydrogen, toluene, 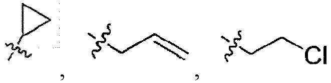 or 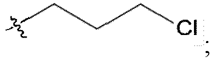 and R₄ is hydrogen or fluorine.

Preferably, R₁ is 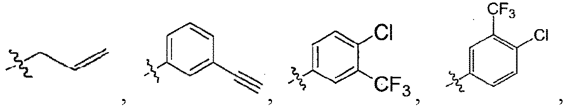 or 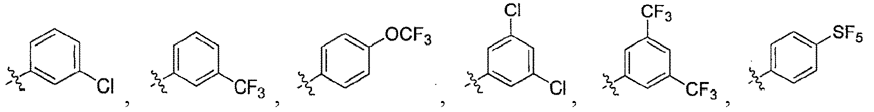 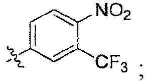 and R₂ is

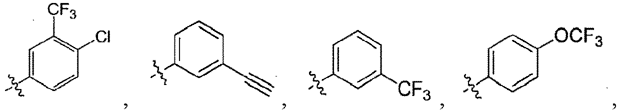

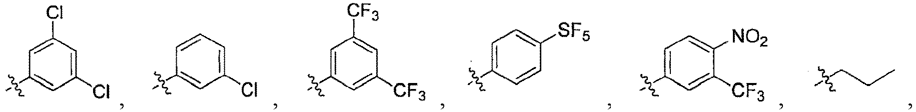

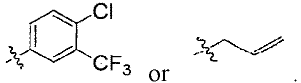

According to some embodiments of the present invention, the amide compound is a urea-containing (carbamide) compound; preferably, it is a diphenylurea analogue. Specifically, the amide compound is a compound having chemical structure (II): 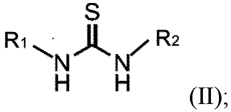 wherein R₁ is 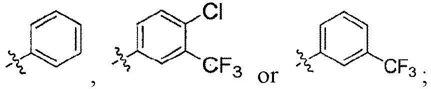 and R₂ is

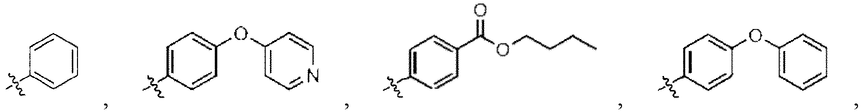

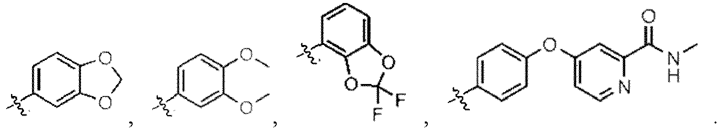

Preferably, the amide compound is a compound having any of chemical structure (III) to (VII):

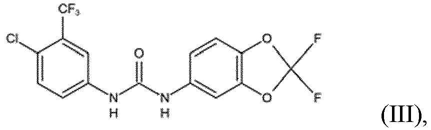

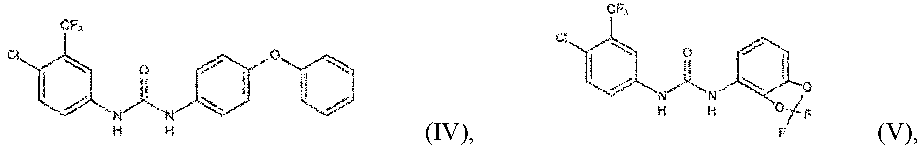

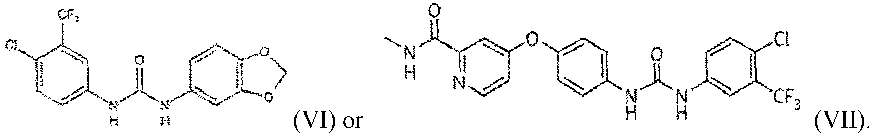

According to some embodiments of the present invention, the fatty acid is a *cis-* monounsaturated fatty acid or a cis-polyunsaturated fatty acid having 16 to 22 carbon atoms. Specifically, the fatty acid has 16, 17, 18, 19, 20, 21 or 22 carbon atoms. Preferably, the fatty acid is a palmitoleic acid, oleic acid, linoleic acid, alpha-linoleic acid, arachidonic acid, eicosapentaenoic acid or docosahexaenoic acid.

According to some embodiments of the present invention, the content of the fatty acid is greater than the content of the amide compound.

### Pharmaceutical compositions and their uses

### Pharmaceutical compositions

A pharmaceutical composition of the present invention includes a foregoing antimicrobial composition and at least one pharmaceutically acceptable carrier. A pharmaceutical composition of the invention may further include a pharmaceutically acceptable excipient or diluent to meet practical needs.

The pharmaceutically acceptable carrier includes a liquid- or solid-state filler, diluent, excipient, solvent, or capsule material and is involved in carrying or delivering an active ingredient from an organ or body part to another organ or body part. The carrier must be "acceptable" in a sense that it is compatible with the other ingredients in the formulation and causes no harm to patients. Some examples of pharmaceutically acceptable carriers are: sugars, such as lactose, glucose, and sucrose; starch, such as corn starch and potato starch; cellulose and its derivatives, such as sodium carboxymethylcellulose, ethyl cellulose, and cellulose acetate; gum tragacanth in powder form; malt; gelatin; talc; excipients, such as cocoa butter and wax for use in suppositories; oils, such as peanut oil, cottonseed oil, safflower seed oil, sesame oil, olive oil, corn oil, and soybean oil; glycols, such as propylene glycol; polyols, such as glycerol, sorbate, mannitol, and polyethylene glycol; esters, such as ethyl oleate and ethyl laurate; agar; buffers, such as magnesium hydroxide and aluminum hydroxide; alginic acid; non-pyrogenic water; isotonic sodium chloride solutions; Ringer's solution; ethanol; phosphate-buffered saline; and other non-toxic biocompatible substances for use in pharmaceutical formulations.

Wetting agents, emulsifiers, and lubricants (e.g., sodium lauryl sulfate and magnesium stearate) may also be present in pharmaceutical compositions of the present invention, and so are colorants, release agents, coating agents, sweeteners, flavoring and perfuming agents, preservatives, and antioxidants.

Some examples of pharmaceutically acceptable antioxidants are: water-soluble antioxidants, such as ascorbic acid, cysteine hydrochloride, sodium bisulfate, sodium metabisulfite, and sodium bisulfite; oil-soluble antioxidants, such as ascorbyl palmitate, butylated hydroxyanisole (BHA), butylated hydroxytoluene (BHT), lecithin, propyl gallate, and α-tocopherol; and metal chelating agents, such as citric acid, ethylenediaminetetraacetic acid (EDTA), sorbitol, tartaric acid, and phosphoric acid.

A pharmaceutical composition of the present invention may be administered orally, nasally, topically, transdermally, transbuccally, sublingually, rectally, intravaginally, and/or parenterally. A pharmaceutical composition of the invention can easily exist in a single-dose form and be prepared by any method well known in the art of pharmacy. The amount of active ingredient that allows the active ingredient and a carrier material to be combined with each other in a single-dose form is usually an amount that produces a therapeutic effect. Generally, this amount of active ingredient accounts for about 1% to about 99%, preferably about 5% to about 70%, and more preferably about 10% to about 30%, of a pharmaceutical composition of the invention.

The method for preparing a pharmaceutical composition of the present invention includes combining an antimicrobial composition of the invention with a carrier and optionally one or more auxiliary ingredients. Generally, a pharmaceutical composition of the invention is prepared by combining an antimicrobial composition of the invention with one or both of a liquid-state carrier and a finely crushed solid-state carrier in an even and closely associated manner and then shaping the resulting product as needed.

For oral administration, a pharmaceutical composition of the present invention may take the form of a capsule, cachet, pill, tablet (with a flavored basis, typically sucrose and gum arabic or gum tragacanth), powder, or granule; or a solution or suspension in an aqueous or non-aqueous liquid; or an oil-in-water or water-in-oil liquid emulsion; or an elixir or syrup; or a pastille (with an inert base such as gelatin and glycerol, or sucrose and gum arabic); and/or a mouthwash, each containing a predetermined amount of an antimicrobial composition of the invention as the active ingredient. A pharmaceutical composition of the invention may also be administered in the form of a bolus, electuary, or paste.

When a pharmaceutical composition of the present invention takes a solid dosage form (e.g., a capsule, tablet, pill, sugar-coated tablet, powder, or granule) to facilitate oral administration, the active ingredient is mixed with one or more pharmaceutically acceptable carriers, such as sodium citrate or dicalcium phosphate and/or any of the following: a filler or extender, such as starch, lactose, sucrose, glucose, mannitol, and/or silicic acid; a binding agent, such as carboxymethyl cellulose, alginate, gelatin, polyvinylpyrolidone, sucrose, and/or gum arabic; a humectant, such as glycerol; a disintegrant, such as agar, calcium carbonate, potato or cassava starch, alginic acid, certain silicates, or sodium carbonate; a solution coagulant, such as paraffin; an absorption promoter, such as a quaternary ammonium compound; a wetting agent, such as cetol or glycerol monostearate; an absorbent, such as kaolin or bentonite; a lubricant, such as talc, calcium stearate, magnesium stearate, solid polyethylene glycol, sodium lauryl sulfate, or a mixture of the above; and a colorant. When in the form of a capsule, tablet, or pill, a pharmaceutical composition of the invention may include a buffer as well. A solid composition of a similar type may also be used as a filler in a hard or soft gelatin-filled capsule that includes an excipient (e.g., lactose or milk sugar and high molecular weight polyethylene glycol).

A tablet can be made by a compressing or molding process, in which one or more optional auxiliary ingredients may be used. A compressed tablet may use a binding agent (e.g., gelatin or hydroxypropyl methylcellulose), lubricant, inert diluent, preservative, disinegrant (e.g., sodium carboxymethyl starch or cross-linked sodium carboxymethylcellulose), surfactant, or dispersant. A molded tablet can be made by molding, in a suitable machine, a mixture of powdery compounds wetted with an inert liquid diluent.

When in the form of a tablet or another solid dosage form (e.g., a sugar-coated tablet, capsule, pill, or granule), a pharmaceutical composition of the present invention may optionally be scored or provided with a coating or shell (e.g., an enteric coating or another coating well known in the art of pharmacy). When taking a solid dosage form, a pharmaceutical composition of the invention may also be formulated with, for example, hydroxypropyl methylcellulose or another polymer matrix, liposome, and/or microspheres in appropriate proportions in order to provide the desired release curve, thereby allowing the active ingredient to be released slowly or in a controlled manner. In addition, when taking a solid dosage form, a pharmaceutical composition of the invention can be sterilized by, for example, being filtered through a bacteria-retaining filter, mixing with a sterilant that is in the form of a solid-state sterilizing composition soluble in sterilized water, or mixing with a sterilizing medium that can be injected into the pharmaceutical composition before the latter is used. A pharmaceutical composition of the invention may also include an optional opalizer and release the active ingredient in an arbitrarily delayed manner only, or preferably, in a specific part of the gastrointestinal tract. Some examples of applicable embedding compositions are polymers and wax. The active ingredient may also take the form of a microcapsule and include one or more of the aforesaid excipients as appropriate.

The liquid dosage forms a pharmaceutical composition of the present invention may take to facilitate oral administration include a pharmaceutically acceptable emulsion, microemulsion, solution, suspension, syrup, and elixir. In addition to the active ingredient, a pharmaceutical composition of the invention may, when in a liquid dosage form, include an inert diluent that is commonly used in the art, such as water or another solvent, solubilizer, or emulsifier, such as ethanol, isopropanol, ethyl carbonate, ethyl acetate, benzyl alcohol, benzyl benzoate, propylene glycol, 1,3-butylene glycol, an oil (in particular cottonseed oil, peanut oil, corn oil, germ oil, olive oil, castor oil, or sesame oil), glycerol, tetrahydrofurfuryl alcohol, a fatty acid ester of polyethylene glycol or sorbitan, or a mixture of the above. Apart from the inert diluent, the orally administrable composition may include an adjuvant, such as a wetting agent, emulsifying and suspending agent, sweetener, favoring agent, colorant, perfume, or preservative. In addition to the active compound, a pharmaceutical composition of the invention may, when in the form of a suspension, include a suspending agent, such as ethoxylated isostearyl alcohol, polyethoxylated sorbitan, sorbitan ester, microcrystalline cellulose, aluminum hydroxide oxide, bentonite, agar, gum tragacanth, or a mixture of the above.

A formulation containing a pharmaceutical composition of the present invention may take the form of a suppository to facilitate rectal or intravaginal administration. Such a formulation can be prepared by mixing one or more compounds of the invention with one or more suitable non-irritant excipients or carriers, wherein the excipients or carriers include, for example, cocoa butter, polyethylene glycol, wax for use in suppositories, and salicylates, are in a solid state when under room temperature and in a liquid state when at body temperature, and will therefore melt, and thereby release the one or more active compounds, in the rectum or vagina. A formulation of the invention for intravaginal administration may also take the form of a pessary, tampon, cream, gel, paste, foam, or spray formulation containing a suitable carrier well known in the art.

The dosage forms a pharmaceutical composition of the present invention may take to facilitate topical or transdermal administration include powder, spray, ointment, paste, cream, lotion, gel, solution, adhesive patch, and inhalant. Under sterilized conditions, an antimicrobial composition of the invention can be mixed with a pharmaceutically acceptable carrier and any preservative, buffer, or propellant that may be required. In addition to the active ingredient, it is feasible for an ointment, paste, cream, or gel to contain an excipient, such as animal fat, plant fat, oil, wax, paraffin, starch, gum tragacanth, a cellulose derivative, polyethylene glycol, silicone, bentonite, silicic acid, talc, zinc oxide, or a mixture of the above.

A transdermal patch has the additional advantage of allowing an antimicrobial composition of the present invention to be delivered to the body in a controlled manner. This dosage form can be produced by dissolving or dispersing an antimicrobial composition of the invention in a suitable medium. The transdermal flux of the compound can be increased by using an absorption enhancer. The flux rate can be controlled by providing a speed-controlling membrane or by dispersing the composition in a polymer matrix or gel.

Ophthalmic formulations, eye ointment, powder, and solutions are also viewed as falling within the scope of the present invention.

To facilitate parenteral administration, a pharmaceutical composition of the present invention may include one or more antimicrobial compositions of the invention and one or more pharmaceutically acceptable sterilized isotonic aqueous or non-aqueous solutions, dispersions, suspensions, emulsions, or sterilized powders that can be reconstituted into a sterile injectable solution or dispersion before use. The one or more pharmaceutically acceptable solutions, dispersions, or the like may contain an antioxidant, buffer, bacteriostat, solute that can make the formulation isotonic with the blood of a test subject, suspending agent, or thickening agent.

Some examples of aqueous and non-aqueous carriers that are suitable for use in pharmaceutical compositions of the present invention are water, ethanol, polyols (e.g., glycerol, propylene glycol, and polyethylene glycol) and suitable polyol mixtures, vegetable oils (e.g., olive oil), and injectable organic esters (e.g., ethyl oleate). Proper fluidity can be maintained by, for example, using a coating material (e.g., lecithin), maintaining the required particle size (in the case of dispersions), or using a surfactant.

A pharmaceutical composition of the present invention may further include an adjuvant, such as a preservative, wetting agent, emulsifier, or dispersant, and various antibacterial and antifungal agents (e.g., paraben, chlorobutanol, and phenol sorbic acid) can be used to ensure prevention of microbial actions. It may also be necessary for a pharmaceutical composition of the invention to include an isotonic agent, such as a sugar or sodium chloride. Moreover, a composition in an injectable pharmaceutical form may include an agent for delaying absorption (e.g., aluminum monostearate or gelatin) so that the composition can be absorbed over a long period of time.

In embodiments where it is desired to prolong the effect of a drug administered by subcutaneous or intramuscular injection, the absorption of the drug can be slowed down by using a liquid suspension of a crystalline or amorphous material with low water solubility. The absorption rate of the drug will then depend on its rate of dissolution, which hinges on the size and form of crystals. Or, a drug for parenteral administration may be dissolved or suspended in an oil-based excipient to achieve delayed absorption.

A pharmaceutical composition of the present invention can be rendered into an injectable depot form by preparing a microcapsule matrix for the composition and forming the matrix in a biodegradable polymer (e.g., polylactide-polyglycolide). The release rate of such a drug can be controlled by adjusting the ratio of the drug to the particular polymer being used and the properties of the polymer. Some other examples of biodegradable polymers are poly(orthoester) and poly(anhydride). An injectable depot formulation may also be prepared by enclosing the drug in a liposome or microemulsion that is compatible with body tissue.

Regardless of the administration route selected, an antimicrobial composition of the present invention (which may be used in a suitable hydrated form) and/or a pharmaceutical composition of the invention can be formulated into a pharmaceutically acceptable dosage form by a conventional method well known to a person skilled in the art.

The actual dosage level of the active ingredient of a pharmaceutical composition of the present invention can be changed in order for the amount of the active ingredient, the composition, and the mode of administration to effectively produce the desired therapeutic response in, and be non-toxic to, a particular patient.

The selected dosage level may depend on various factors, including the activity of the particular compound of the present invention being used; the activity of the ester, salt, or amide in the particular compound being used; the route of administration; the time of administration; the excretion rate of the particular compound being used; the duration of treatment; the drugs, compounds, and/or materials used in combination with the particular compound being used; and the age, gender, body weight, conditions, general health, and prior medical history of the patient being treated, among other factors well known in the art of pharmacy.

A physician or veterinarian of ordinary skill in the art can rapidly determine and prescribe the effective amount of the pharmaceutical composition required. For example, the physician or veterinarian may start by making the dosage level of the compound of the present invention used in the pharmaceutical composition lower than that required for producing the desired therapeutic effect, and then gradually increase the dosage until the desired effect is obtained.

### Use

The present invention further provides a use of the above-mentioned antimicrobial composition in preparing a drug for inhibiting the growth of microbial cells and microbial biofilms.

According to some embodiments of the present invention, the microbial cells are cells of human pathogenic bacteria. Specifically, the human pathogenic bacteria is selected from a group consisting of *Staphylococcus aureus* (*S. aureus*)*, Staphylococcus haemolyticus* (*S. haemolyticus*)*, Staphylococcus hominis* (*S. hominis*), *Staphylococcus intermedius* (*S. intermedius*)*, Staphylococcus saprophyticus* (*S. saprophyticus*)*, Staphylococcus lugdunesis* (*S. lugdunesis*)*, Erysipelothrix rhusiopathiae, Enterococcus faecalis*, *Enterococcus faecium, VR-E. faecium, Bacillus cereus, Bacillus subtilis, Corynebacterium diphtheriae, Listeria monocytogenes, Streptococcus pyogenes, Clostridium difficile, Escherichia coli, Salmonelia Typhimurium, Acinetobacter baumannii, Mycobacterium tuberculosis,* methicillin-resistant *Staphylococcus aureus* (MRSA) and vancomycin-resistant *Staphylococcus aureus* (VRSA).

According to some embodiments of the present invention, the microbial biofilm is the biofilm of human pathogenic bacteria or fungi. Specifically, the human pathogenic bacteria or fungi is selected from a group consisting of *Staphylococcus haemolyticus, Klebsiella pneumoniae, Pseudomonas aeruginosa, Escherichia coli, Enterococcus faecalis*, *Enterococcus faecium, Candida albicans* and *Staphylococcus aureus.*

### Examples

In this section, the invention is described in detail by means of the following examples of embodiments. These examples are for illustrative purposes only, and the various modifications and variations are readily apparent to those with ordinary knowledge in the art to which the invention belongs. Accordingly, the various embodiments of the invention will be described in detail below, but the invention is not limited to those embodiments listed in this specification.

### Materials and Methods

### Cells

The murine macrophage cell line RAW264.7 (BCRC) and human cervical epithelioid carcinoma cell line HeLa (BCRC) were maintained in Dulbecco's modified Eagle's medium (DMEM; Gibco-BRL) supplemented with 10% heat-inactivated FBS (Gibco-BRL). The human embryonic kidney cell line HEK-293 (BCRC) and the immortalized nontumorigenic keratinocyte cell line HaCaT (obtained from Dr. Yung-Chiy Chang, National Taiwan University, Taiwan) were maintained in Roswell Park Memorial Institute (RPMI; Gibco-BRL) medium supplemented with 10% heat-inactivated FBS. Cells were cultured in T-75 flasks at 37°C in a 5% CO₂ atmosphere.

### Reagents

Samples of acylamine compounds were synthesized and dissolved in DMSO to produce a stock solution (10 g/L). Aminobenzylpenicillin (10 g/L; USB), benzocillin (10 g/L; Sigma-Aldrich), ofloxacin (1 g/L; Bio Basic) and vancomycin (10 g/L; GoldBio) were dissolved in sterilized deionized water to produce stock solutions. Arachidonic acid, eicosapentaenoic acid, elaidic acid, erucic acid, gondoic acid, α-linolenic acid, linoleic acid, myristoleic acid, nervonic acid, oleic acid, palmitoleic acid, and stearic acid were purchased from Cayman Chemical and were dissolved in 95% ethanol to prepare stock solutions (10 g/L). Docosahexaenoic acid (DHA, 40 g/L; Santa Cruz), erythromycin (10 g/L; Bio Basic) and tetracycline (10 g/L; Bio Basic) were dissolved in 95% ethanol to make stock solutions. Ciprofloxacin (10 g/L; Sigma-Aldrich), daptomycin (10 g/L; Sigma-Aldrich), linezolid (10 g/L; Santa Cruz), rifampicin (10 g/L; Bio Basic) and sorafenib (10 g/L; kindly provided by Bayer Pharmaceuticals, Inc.) were dissolved in DMSO to produce stock solutions.

### Antibacterial activity assay

The antimicrobial activity was based on MIC and MBC; MIC is the minimum inhibitory concentration and MBC is the minimum bactericidal concentration. The MIC of individual test agents against MRSA USA300 strains was determined using the broth microdilution method according to CLSI guidelines. Briefly, overnight bacterial cultures in LB medium were diluted 1:50 in fresh LB medium and incubated at 37°C until reaching an OD₆₀₀ of 0.5 to 0.6. Bacterial suspensions were diluted to a final concentration of 5×10⁵ cfu/mL in fresh CAMHB and then exposed to each test agent at increasing doses in triplicate in 96-well plates for 24 hours at 37°C. The MIC of each agent was defined as the lowest concentration without visible bacterial growth. The MBC was determined by subculturing 5 µL of broth dilutions at or above the MIC on CAMHB agar plates for 24 hours at 37°C. The lowest broth dilution that had no viable bacteria was considered the MBC.

To evaluate the effect of FAs on the antibacterial activities of sorafenib, SC5005 and PK150, bacterial suspensions were diluted to a final concentration of 5×10⁵ cfu/mL in fresh CAMHB supplemented with designated concentrations of FAs, followed by using the protocols described above to determine the MICs and MBCs of individual agents. Then, the combination effect was measured by calculating their corresponding fractional inhibitory concentration index (FICI) with the equation ΣFIC (FICi) = FIC_{A} + FIC_{B} = (Comb_{A}/MIC_{A}) + (Comb_{B}/MIC_{B}), where MIC_{A} and MIC_{B} are the MICs of drugs A and B alone, respectively, and Comb_{A} and Comb_{B} are the concentrations of drugs A and B in combination, respectively. Combination effect was defined as an FICI ≤ 0.5, no interaction was defined as an FICI > 0.5 - 4.0, and antagonism was defined as an FICI > 4.

### Antiproliferation assay

The effects of SC5005 and DHA on the viability of mammalian cells were assessed using a 3-(4,5-dimethylthiazol-2-yl)-2,5-diphenyl-2H-tetrazolium bromide (MTT) assay in six replicates per treatment group. Briefly, cells were seeded into 96-well, flat-bottomed plates at 10⁴ cells/well, cultured for 24 hours, and then exposed to SC5005 at concentrations ranging from 5-30 mg/L or the combination of SC5005 with designated concentrations of DHA for 24 hours. Controls were treated with DMSO and ethanol vehicles at concentrations equal to those for drug-treated cells. At the end of treatments, the medium was replaced with 0.5 g/L MTT-containing culture medium, and the cells were incubated in a CO₂ incubator at 37°C for an additional 1.5 hours. Supernatants were removed from the wells, and the reduced MTT dye was solubilized in DMSO followed by measuring the absorbance at 570 nm of each well using a VersaMax microplate reader (Molecular Devices, USA).

### Persister killing assay

Log-phase bacteria were adjusted to OD₆₀₀ = 0.2 and then treated with vancomycin at 16× MIC for 12 hours at 37°C. Bacteria were collected by centrifugation at 4000× g for 10 min at 4°C, washed with ice-cold PBS, and suspended in PBS with test agents. Controls received DMSO and ethanol vehicles at concentrations equal to those for drug-treated bacteria. At the designated times, the number of viable bacteria was assessed by serial dilution of a 100 µL aliquot bacterial suspension in PBS and then spread onto LB agar plates. After incubation at 37°C for 18-24 hours, the number of colonies was counted and expressed as cfu/mL.

### Biofilm eradication assay

The eradication activity of daptomycin, vancomycin, experimental acyl compounds and experimental acyl compounds along with fatty acids against biofilms of microorganisms was evaluated as described above. Briefly, bacteria were grown in tryptic soy broth (Becton Dickinson, USA) and biofilms were formed on the pegs of the lid of a 96-well dish immersed in bacterial cultures at 5x10⁵ cfu/mL. After 24 hours, the pegs were washed twice with PBS and then transferred to a new 96-well dish containing test reagents at concentrations ranging from 0.125 mg/L to 1024 mg/L in each well. After incubation at 37°C for 24 hours, the peg-lids were washed and placed onto a new 96-well plate with fresh CAMHB in each well and sonicated for 5 minutes to disrupt the biofilm on the pegss, followed by a cfu assay to assess the number of viable bacteria in each well. To determine the minimum biofilm removal concentration, the 96-well plates were covered with new lids and incubated at 37°C for 24 hours, and then the viable bacteria in each well were counted using the cfu assay.

### Statistical analysis

The data are expressed as the mean ± SD. Differences among group means were calculated using Prism software (v6.0, GraphPad, USA) using one-way ANOVA with Dunnett's comparison and were considered significant at a P value <0.05.

### Results

### Combination effect of the amide compound with each of the fatty acids

The amide compound used in this experiment is SC5005 (whose structure is shown below). SC5005 was combined with each of a number of different fatty acids, before the antibacterial activity of SC5005 against the Methicillin-resistant *Staphylococcus aureus* (MRSA) USA300 strain was determined. The results are presented in Table 1 and Table 2, which respectively show the minimum inhibitory concentration (MIC) and minimal bactericidal concentration (MBC) of SC5005 against MRSA USA300 after combination with the different fatty acids.

The structure of SC5005:

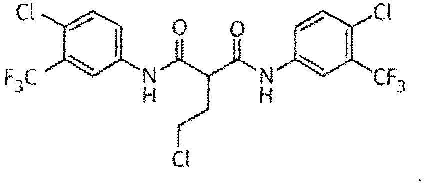

**Table 1**

| fatty acid (mg/L) | MIC (mg/L) | | | | | | | |
|---|---|---|---|---|---|---|---|---|
| | fatty acid | SC5005 (combination effect) | | | | | | |
| | | 0 | 4 | | 8 | | 16 | |
| myristic acid (C14:1n5c) | 512 | 0.25 | 0.25 | (NI) | 0.25 | (NI) | 0.25 | (NI) |
| palmitoleic acid (C16:1n7c) | 64 | 0.25 | 0.0625 | (Syn) | 0.0312 5 | (Syn) | 0.03125 | (Syn) |
| stearic acid (C18:0) | 512 | 0.25 | 0.25 | (NI) | 0.25 | (NI) | 0.25 | (NI) |
| elaidic acid (C18:1n9t) | > 1024 | 0.25 | 0.25 | (NI) | 0.25 | (NI) | 0.25 | (NI) |
| oleic acid (C18:1n9c) | 512 | 0.25 | 0.03125 | (Syn) | 0.0312 5 | (Syn) | 0.03125 | (Syn) |
| linoleic acid (C18:2n6c) | 1024 | 0.25 | 0.0156 | (Syn) | 0.0078 | (Syn) | 0.0078 | (Syn) |
| α- linolenic acid (C18:3n3c) | 256 | 0.25 | 0.0625 | (Syn) | 0.0625 | (Syn) | 0.0156 | (Syn) |
| gondoic acid (C20:1n9c) | 512 | 0.25 | 0.125 | (NI) | 0.25 | (NI) | 0.25 | (NI) |
| arachidonic acid (C20:4n6c) | 128 | 0.25 | 0.0625 | (Syn) | 0.0625 | (Syn) | 0.03125 | (Syn) |
| Eicosapentaenoic acid (C20:5n3c) | 64 | 0.25 | 0.0625 | (Syn) | 0.0625 | (Syn) | 0.03125 | (Syn) |
| erucic acid (C22:1n9c) | 128 | 0.25 | 0.125 | (NI) | 0.25 | (NI) | 0.25 | (NI) |
| docosahexaenoic acid (C22:6n3c) | 1024 | 0.25 | 0.03125 | (Syn) | 0.0000 15 | (Syn) | 0.00000 38 | (Syn) |
| nervonic acid (C24:1n9c) | 128 | 0.25 | 0.25 | (NI) | 0.25 | (NI) | 0.25 | (NI) |

**Table 2**

| fatty acid (mg/L) | MBC (mg/L) | | | | | | | |
|---|---|---|---|---|---|---|---|---|
| | fatty acid | SC5005 (combination effect)^{b} | | | | | | |
| | | 0 | 4 | | 8 | | 16 | |
| myristic acid (C14:1n5c) | >1024 | 2 | 1 | (NI) | 1 | (NI) | 1 | (NI) |
| palmitoleic acid (C16:1n7c) | 512 | 2 | 0.0625 | (Syn) | 0.03125 | (Syn) | 0.03125 | (Syn) |
| stearic acid (C18:0) | >1024 | 2 | 2 | (NI) | 2 | (NI) | 2 | (NI) |
| elaidic acid (C18:1n9t) | >1024 | 2 | 1 | (NI) | 1 | (NI) | 0.5 | (NI) |
| oleic acid (C18:1n9c) | >1024 | 2 | 0.125 | (Syn) | 0.0625 | (Syn) | 0.0625 | (Syn) |
| linoleic acid (C18:2n6c) | >1024 | 2 | 0.0625 | (Syn) | 0.03125 | (Syn) | 0.03125 | (Syn) |
| α- linolenic acid (C18:3n3c) | 1024 | 2 | 0.5 | (Syn) | 0.25 | (Syn) | 0.125 | (Syn) |
| gondoic acid (C20:1n9c) | >1024 | 2 | 0.125 | (Syn) | 0.25 | (Syn) | 0.25 | (Syn) |
| arachidonic acid (C20:4n6c) | 1024 | 2 | 0.5 | (Syn) | 0.0625 | (Syn) | 0.03125 | (Syn) |
| eicosapentaenoic acid (C20:5n3c) | 1024 | 2 | 1 | (Syn) | 0.5 | (Syn) | 0.0625 | (Syn) |
| erucic acid (C22:1n9c) | 1024 | 2 | 0.25 | (Syn) | 0.25 | (Syn) | 0.25 | (Syn) |
| docosahexaenoic /DHA (C22:6n3c) | 1024 | 2 | 0.03125 | (Syn) | 0.0078 | (Syn) | 0.0039 | (Syn) |
| nervonic acid (C24:1n9c) | >1024 | 2 | 2 | (NI) | 2 | (NI) | 2 | (NI) |

In Table 1 and Table 2, the structure of individual fatty acids is represented by the form CA:DnEF, where A is the number of carbon atoms, D is the number of double bonds, E is the number of carbons from the methyl end to the first carbon in the double bond closest to the methyl end and F denotes the geometric property of double bonds (c: cis or t: trans). The combination effect was measured by calculating the corresponding fractional inhibitory concentration index (FICI). Synergy (Syn) was defined as an FICI ≤0.5, no interaction (NI) was defined as an FICI > 0.5 and < 4.0, and antagonism (Ant) was defined as an FICI > 4.

As can be seen in Table 1 and Table 2, SC5005 and cis-unsaturated fatty acids exhibited synergy. By contrast, although saturated stearic acid (C18:0) has the same carbon chain length as oleic acid, SC5005 and saturated stearic acid failed to exhibit synergy against MRSA USA300; elaidic acid (C18:1n9t) also has 18 carbon atoms, but it has a trans double bond as well and therefore did not exhibit synergy with SC5005. It can be inferred from the above that a cis double bond is important to the synergistic antibacterial effect of an amide compound and a fatty acid. Moreover, the results in Table 1 and Table 2 show that the combination of SC5005 and palmitoleic acid (C16:1n7c), which is a cis-monounsaturated fatty acid with 16 carbon atoms, exhibited synergy; and that gondoic acid (C20:1n9c) and erucic acid (C22:1n9c), which are cis-monounsaturated fatty acids with 20 and 22 carbon atoms respectively, each exhibited a synergistic bactericidal effect with SC5005 but had no effect on the MIC of SC5005. In addition, neither of nervonic acid (C24:1n9c) and myristic acid (C14:1n5c), which have 24 and 14 carbon atoms respectively, showed any synergistic activity with SC5005. Hence, it can be inferred that SC5005 and a cis-unsaturated fatty acid whose carbon chain has 16-22 carbon atoms can produce a synergistic antibacterial effect.

Furthermore, the antibacterial activity of the combination of SC5005 and linoleic acid (C18:2n6c) is higher than that of the combination of SC5005 and oleic acid (C18:1n9c) and the combination of SC5005 and α-linolenic acid (C18:3n3c). Also, despite their difference in the number of double bonds, arachidonic acid (C20:4n6c) and eicosapentaenoic acid (EPA; C20:5n3c) had the same effect on the antibacterial activity of SC5005. It can therefore be inferred that the number of double bonds in the carbon chain of an unsaturated fatty acid has nothing to do with the synergistic antibacterial activity of the combination of the fatty acid and SC5005.

### Synergy of the fatty acid and each of the amide compounds

In this section, each of the various amide compounds was combined with a fatty acid of a fixed concentration, and the change in antibacterial activity of each amide compound against the MRSA USA300 strain was observed, as shown in Table 3. The fatty acid used herein is docosahexaenoic acid (DHA).

**Table 3**

| | | | | **+ 8 mg/L DHA** | |
|---|---|---|---|---|---|
| | | **MIC (mg/L)** | **MBC (mg/L)** | **MIC (mg/L)** | **MBC (mg/L)** |
| Sorafenib | 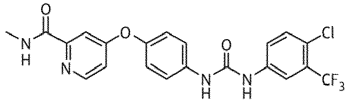 | >64 | >64 | 0.5 | 1 |
| SC5005 | 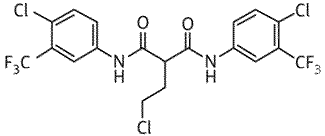 | 0.25 | 2 | 0.0000038 | 0.0078 |
| SC5010 | 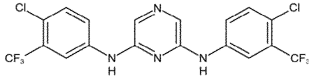 | >64 | >64 | >64 | >64 |
| SC5015 | 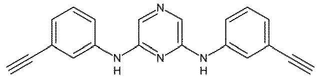 | >64 | >64 | >64 | >64 |
| SC5020 | 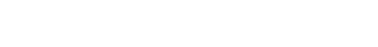 | >64 | >64 | 0.125 | 0.125 |
| SC5021 | | >64 | >64 | 64 | 64 |
| SC5022 | | >64 | >64 | >64 | >64 |
| SC5023 | | >64 | >64 | 16 | 16 |
| SC5024 | | >64 | >64 | 8 | 8 |
| SC5025 | | 16 | 32 | 2 | 2 |
| SC5026 | | >64 | >64 | >64 | >64 |
| SC5027 | | >64 | >64 | 64 | >64 |
| SC5032 | 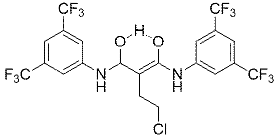 | 8 | 32 | 0.5 | 0.5 |
| SC5033 | 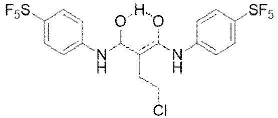 | 64 | 64 | 2 | 2 |
| SC5034 | | 16 | 32 | 1 | 1 |
| SC5035 | | 16 | 32 | 1 | 2 |
| SC5037 | | 1 | 2 | 0.0039 | 0.0078 |
| SC5038 | | >64 | >64 | >64 | >64 |
| SC5040 | | 1 | 2 | 0.0039 | 0.125 |
| SC5041 | | 0.5 | 2 | 0.0625 | 0.0625 |
| SC5042 | 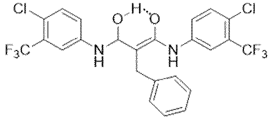 | 2 | 16 | 0.125 | 0.125 |
| SC5043 | | 2 | 4 | 0.0156 | 0.0156 |
| SC5044 | | 0.5 | 2 | 0.000975 | 0.0078 |
| SC5045 | 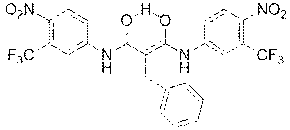 | 0.25 | 1 | 0.0625 | 0.0625 |
| SC5048 | | >64 | >64 | >64 | >64 |
| SC5049 | | >64 | >64 | 8 | 8 |
| SC5050 | | >64 | >64 | 8 | 16 |
| SC5052 | | >64 | >64 | 1 | 2 |
| SC5053 | | >64 | >64 | 1 | 2 |
| SC5178 | 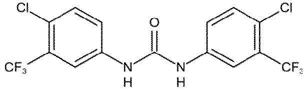 | 0.25 | 2 | 0.00012 | 0.00156 |
| SC5101 | 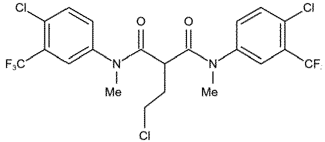 | >64 | >64 | >64 | >64 |
| SC5102 | 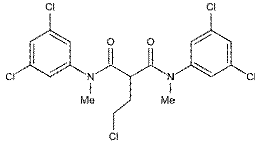 | >64 | >64 | >64 | >64 |
| SC5103 | 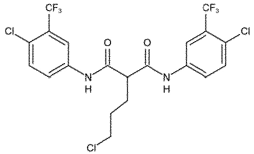 | 0.25 | 2 | 0.0039 | 0.0078 |
| SC5104 | 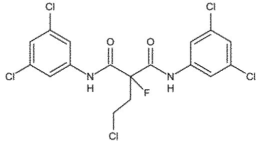 | 0.5 | 4 | 0.0078 | 0.0078 |
| SC5105 | 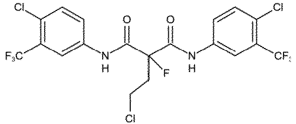 | >64 | >64 | 0.125 | 0.125 |
| SC5106 | 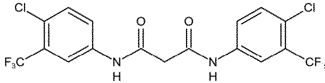 | >64 | >64 | 0.25 | 0.5 |
| SC5107 | 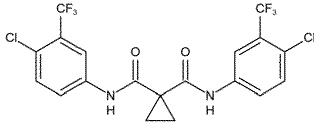 | >64 | >64 | 0.0625 | 0.125 |
| PK150 | 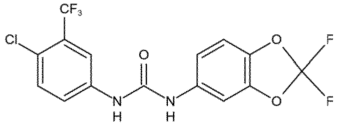 | 0.0625 | 2 | 0.00049 | 0.00098 |
| 3-006 | 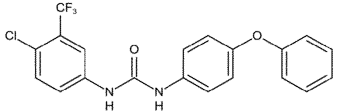 | 0.125 | 1 | 0.0078 | - |
| 1-164 | 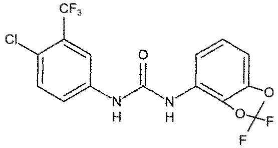 | 0.25 | 4 | 0.03125 | - |
| 1-159 | 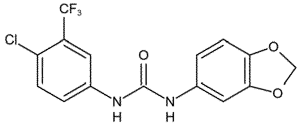 | 32 | 64 | 0.125 | - |

As can be seen in Table 3, several samples of amide compounds have synergistic antibacterial activity with DHA (i.e., the MIC after adding DHA is less than 1/4 of its original MIC), including sorafenib, PK150 and its analogues.

### Antibacterial activity of some compositions of the present invention against antibiotic-resistant Staphylococcus aureus

The compositions employed in this experiment used Sorafenib, SC5005, and PK150 as their respective amide compounds and DHA as their fatty acid. The antibacterial activity of the SC5005-DHA composition against a series of antibiotic-resistant *Staphylococcus aureus* was analyzed, and the results are shown in Table 4.

**Table 4**

| Bacterial strain | Resistance pattern | MIC (mg/L) | | | | | | | | | |
|---|---|---|---|---|---|---|---|---|---|---|---|
| | | DHA | Sorafenib | SC 5005 | PK-15 0 | + 8 mg/L DHA (combination effect) | | | | | |
| | | | | | | Sorafenib | | SC5005 | | PK150 | |
| *S. aureus* ATCC29213 | - | 1024 | 64 | 0.25 | 0.0625 | 0.5 | (Syn) | 0.0039 | (Syn) | 0.0039 | (Syn) |
| *S. aureus* NCTC8325 | - | 1024 | 4 | 0.25 | 0.0625 | 0.5 | (Syn) | 0.00000 19 | (Syn) | 0.00003 5 | (Syn) |
| MRSA USA300 | AMP, ERY, OXA, OFX, CIP | 1024 | >64 | 0.25 | 0.0625 | 0.5 | (Syn) | 0.00001 5 | (Syn) | 0.00049 | (Syn) |
| MRSA ATCC33591 | AMP, ERY, OXA, TET, | 1024 | >64 | 0.25 | 0.0625 | 0.5 | (Syn) | 0.00006 1 | (Syn) | 0.00006 1 | (Syn) |
| MRSA ATCC33592 | AMP, ERY, OXA, TET, RIF | 1024 | >64 | 0.25 | 0.0625 | 0.5 | (Syn) | 0.00049 | (Syn) | 0.00098 | (Syn) |
| MRSA ATCC43300 | AMP, ERY, OXA | 1024 | >64 | 0.25 | 0.0625 | 0.5 | (Syn) | 0.00024 | (Syn) | 0.00098 | (Syn) |
| MRSA ATCC49476 | AMP, ERY, OXA, TET | 1024 | >64 | 0.25 | 0.0625 | 0.5 | (Syn) | 0.00000 095 | (Syn) | 0.00001 5 | (Syn) |
| MRSA SCCmec V_{T} | AMP, ERY, OXA, OFX | 1024 | >64 | 0.25 | 0.125 | 0.5 | (Syn) | 0.00000 19 | (Syn) | 0.00098 | (Syn) |
| VRSA SJC1200 | VAN | 1024 | >64 | 0.25 | 0.125 | 0.5 | (Syn) | 0.00000 19 | (Syn) | 0.00012 | (Syn) |
| *S.aureus* DAP-NS | AMP, ERY, TET, OFX, CIP | 1024 | 32 | 0.25 | 0.0625 | 0.5 | (Syn) | 0.00000 19 | (Syn) | 0.0156 | (Syn) |

As can be seen in Table 4, AMP refers to ampicillin; ERY refers to erythromycin; OXA refers to oxacillin; TET refers to tetracycline; OFX refers to ofloxacin; CIP refers to ciprofloxacin; LZD refers to linezolid; RIF refers to rifampicin; RIF refers to ciprofloxacin; VAN refers to vancomycin.

It can be seen in Table 4 that, despite their resistance to various antibiotics, all the bacterial strains under the examination exhibited the same level of sensitivity to DHA alone and to SC5005 alone, the corresponding MICs being 1024 mg/L and 0.25 mg/L respectively. By contrast, even though each bacterial strain had different sensitivity to the SC5005-DHA composition, the results in general show that the SC5005-DHA composition produced a synergistic antibacterial effect on all the strains under test.

### Cytotoxicity of a composition of the present invention to mammalian cells

The composition employed in this experiment used SC5005 as its amide compound and DHA as its fatty acid. The antiproliferative activity of SC5005 toward four different mammalian cell lines was evaluated at a series of increasing DHA concentrations, and the results are shown in Table 5. The mammalian cell lines are murine macrophages (RAW264.7), human cervical cancer epithelial cells (HeLa), human keratinocytes (HaCaT), and human embryonic kidney cells (HEK-293). As can be seen in Table 5, there was no significant change in the cytotoxicity of SC5005 to each cell line even when the DHA concentration reached 16 mg/L. It can therefore be inferred that the synergy exhibited by a composition of the invention is directed to a pathogen and will not cause damage to mammalian cells.

**Table 5**

| DHA (mg/L) | CC₅₀ of SC5005 (mg/L) | | | |
|---|---|---|---|---|
| | RAW264.7 | HeLa | HaCaT | HEK-293 |
| 0 | 19.5 ± 0.6 | 22.5 ± 0.7 | 18.0 ± 1.7 | 14.1 ± 1.4 |
| 4 | 22.6 ± 0.8 | 24.2 ± 3.6 | 19.5 ± 1.7 | 15.6 ± 0.6 |
| 8 | 19.7 ± 2.5 | 21.9 ± 1.6 | 20.5 ± 0.3 | 14.9 ± 1.6 |
| 16 | 21.2 ± 2.3 | 21.4 ± 1.4 | 18.1 ± 1.6 | 14.0± 0.8 |

### Elimination of persister cells by a composition of the present invention

In this experiment, the inventor analyzed the time-kill kinetics of SC5005, DHA, and a combination thereof against *Staphylococcus aureus* NCTC8325 and MRSA USA300, and the results are shown in FIG. 1. It can be seen in FIG. 1 that SC5005 killed more than 99.9% of the bacteria within 24 hours. By contrast, 8 mg/L DHA exhibited only a moderate inhibitory effect on the growth of *Staphylococcus aureus* NCTC8325 in the initial stage of the treatment; after that, the growth of the bacteria rebounded to the same degree as that of the control group. It is worth noting that the combination of SC5005 and DHA eliminated all the bacteria within 10 minutes, indicating that the synergistic antibacterial activity of SC5005 and DHA resulted in a very strong bactericidal effect. Besides, it is well known in the art that *Staphylococcus aureus* can enter a dormant state (i.e., become the so-called persister cells) and develop resistance to antibiotic treatment, thus leading to repeated bacterial infections. So, in order to separate the *Staphylococcus aureus* persister cells, we treated *Staphylococcus aureus* in phosphate-buffered saline with vancomycin, an antibiotic, at a bactericidal concentration for 12 hours to eliminate non-persister cells, and as shown in FIG. 1, the remaining bacteria cells were resistant to the killing activity of vancomycin and daptomycin, another bactericidal antibiotic, meaning this bacterial cell subgroup was composed of persister cells. FIG. 1 also shows that SC5005 and its combination with DHA were very effective in killing persister cells: the persister cells were eradicated by SC5005 and the combination of SC5005 and DHA within 24 hours and 30 minutes respectively. This finding indicates that the mechanism of action (MoA) of the combination of SC5005 and DHA has nothing to do with the metabolic activity of bacteria.

### Removal of Staphylococcus aureus biofilms by a composition of the present invention

*Staphylococcus aureus* can form a biofilm when attached to a medical implant or host tissue, and biofilms are generally associated with chronic infection and treatment failure because they provide an environment advantageous to the formation of persister cells. To verify that SC5005 and DHA can eradicate *Staphylococcus aureus* biofilms, the inventor treated bacterial biofilms with SC5005 either alone at a series of increasing concentrations or in combination with 8 mg/L DHA for 24 hours, and the results are shown in FIG. 2. According to FIG. 2, SC5005 exhibited superior biofilm removing activity in comparison with daptomycin and vancomycin, and the 8 mg/L DHA not only failed to enhance the biofilm removing activity of SC5005, but even produced an antagonistic effect when it came to MRSA USA300 biofilms, indicating that the biofilm matrix, which is composed essentially of polysaccharides, proteins, and nucleic acids, may interfere with the synergy of SC5005 and DHA. When the DHA concentration was subsequently increased to 80 mg/L, it was observed that the biofilm removing activity of SC5005 was increased 32 times. Moreover, as can be seen in FIG. 2, the combination of 1 mg/L SC5005 and 80 mg/L DHA removed nearly 99% of the bacteria in biofilms within one minute and did not produce detectable cytotoxicity to RAW264.7 for as long as 60 minutes. Generally speaking, the combinations of SC5005 and DHA showed superior and more efficient bactericidal activity against *Staphylococcus aureus* biofilms than second-line antibiotics.

## Claims

1. An antimicrobial composition for use in the treatment of bacterial infection for inhibiting the growth of a microbial cell and the growth of a microbial biofilm, wherein the antimicrobial composition comprises:
an amide compound; and
a fatty acid;
wherein the fatty acid is a cis-unsaturated fatty acid with 16 to 22 carbon atoms; and
the amide compound is:
(a) a compound having chemical structure (I-1) or (I-2): 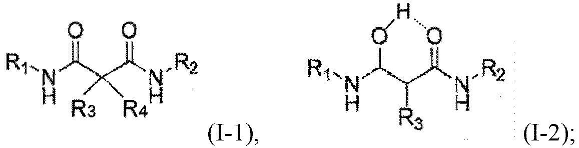 where each of R₁ and R₂ is 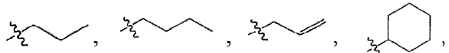 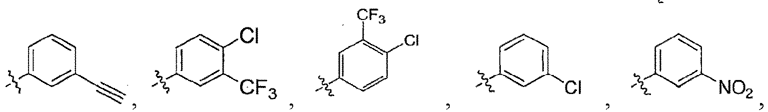 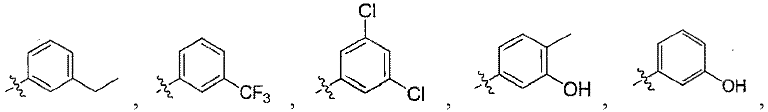 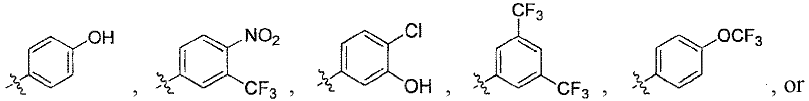 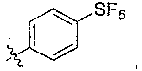 and R₁ and R₂ are not concurrently 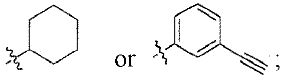 R₃ is hydrogen, toluene, 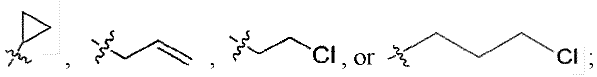 and R₄ is hydrogen or fluorine; or
(b) a compound having chemical structure (II): 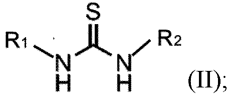 where R₁ is 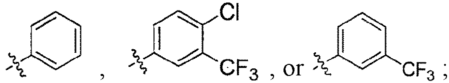 and R₂ is 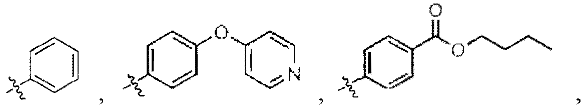 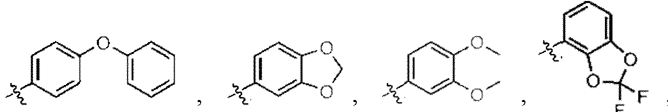 , or

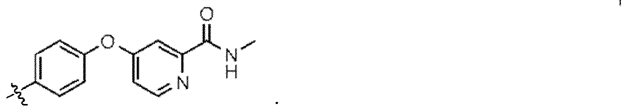

2. The antimicrobial composition for use in the treatment of bacterial infection of claim 1, wherein the amide compound is according to case (a) and R₁ is 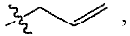 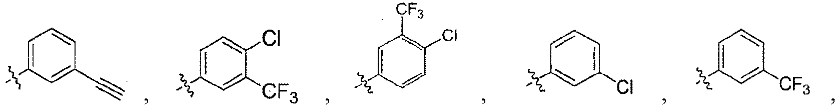 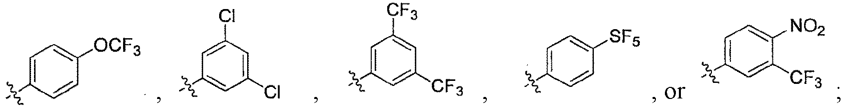 and R₂ is

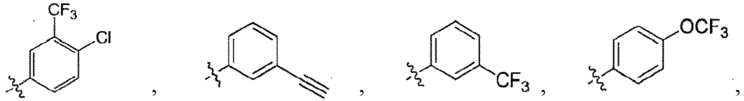

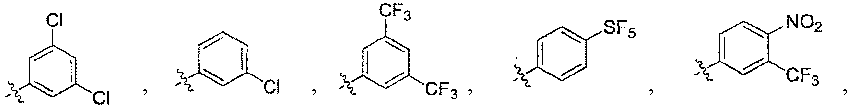

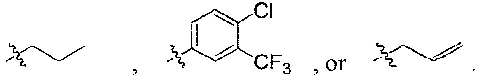

3. The antimicrobial composition for use in the treatment of bacterial infection of claim 1, wherein the amide compound is a compound having any of chemical structures (III) to (VII):

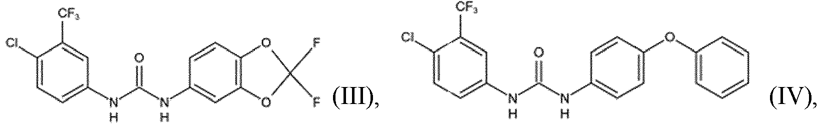

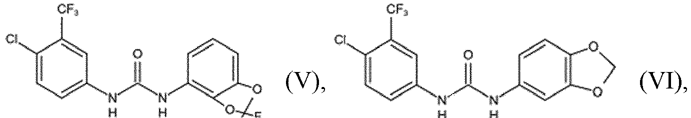

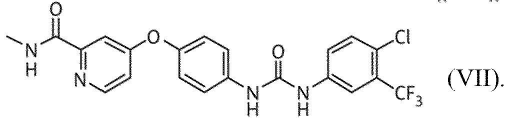

4. The antimicrobial composition for use in the treatment of bacterial infection of claim 1, wherein the fatty acid is a cis-monounsaturated fatty acid or cis-polyunsaturated fatty acid with 16 to 22 carbon atoms.

5. The antimicrobial composition for use in the treatment of bacterial infection of claim 1, wherein the fatty acid is palmitoleic acid, oleic acid, linoleic acid, α-linolenic acid, arachidonic acid, eicosapentaenoic acid, or docosahexaenoic acid.

6. The antimicrobial composition for use in the treatment of bacterial infection of claim 1, wherein the content of the fatty acid is greater than the content of the amide compound.

7. The antimicrobial composition for use in the treatment of bacterial infection of any of the preceding claims, wherein the microbial cell is a cell of a human pathogenic bacterium.

8. The antimicrobial composition for use in the treatment of bacterial infection of claim 7, wherein the human pathogenic bacterium is selected from a group consisting of *Staphylococcus aureus (S. aureus), Staphylococcus haemolyticus (S. haemolyticus), Staphylococcus hominis (S. hominis), Staphylococcus intermedius (S. intermedius), Staphylococcus saprophyticus (S. saprophyticus), Staphylococcus lugdunesis (S. lugdunesis), Erysipelothrix rhusiopathiae, Enterococcus faecalis*, *Enterococcus faecium, VR-E. faecium, Bacillus cereus, Bacillus subtilis, Corynebacterium diphtheriae, Listeria monocytogenes, Streptococcus pyogenes, Clostridium difficile, Escherichia coli, Salmonelia Typhimurium, Acinetobacter baumannii, Mycobacterium tuberculosis,* methicillin-resistant *Staphylococcus aureus* (MRSA) and vancomycin-resistant *Staphylococcus aureus* (VRSA).

9. The antimicrobial composition for use in the treatment of bacterial infection of any of claims 1 to 6, wherein the microbial biofilm is a biofilm of a human pathogenic bacterium or fungus.

10. The antimicrobial composition for use in the treatment of bacterial infection of claim 9, wherein the human pathogenic bacterium or fungus is selected from a group consisting of *Staphylococcus haemolyticus, Klebsiella pneumoniae, Pseudomonas aeruginosa, Escherichia coli, Enterococcus faecalis*, *Enterococcus faecium, Candida albicans* and *Staphylococcus aureus.*

11. A pharmaceutical composition, comprising the antimicrobial composition of any of claims 1 to 10 and at least one pharmaceutically acceptable carrier.

## Patentansprüche

1. Eine antimikrobielle Zusammensetzung zur Verwendung bei der Behandlung bakterieller Infektionen zur Hemmung des Wachstums einer mikrobiellen Zelle und des Wachstums eines mikrobiellen Biofilms, wobei die antimikrobielle Zusammensetzung umfasst:
eine Phenylharnstoffverbindung; und
eine Fettsäure;
wobei die Fettsäure eine cis-ungesättigte Fettsäure mit 16 bis 22 Kohlenstoffatome ist; und
die Phenylharnstoffverbindung ist:
(a) eine Verbindung mit der chemischen Struktur (I-1) oder (I-2): 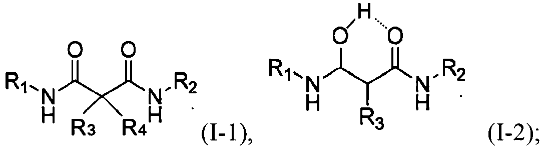 wobei jedes von R₁ und R₂ 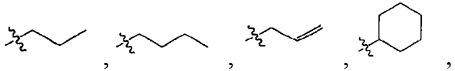 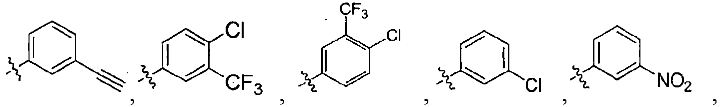 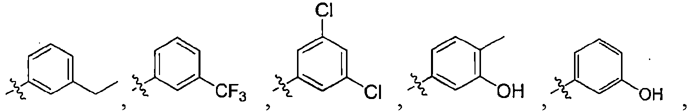 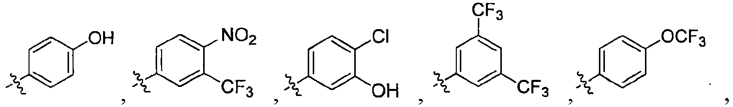 oder 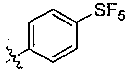 und R₁ und R₂ sind nicht gleichzeitig 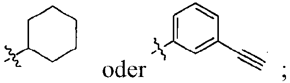 R₃ Wasserstoff, Toluol, 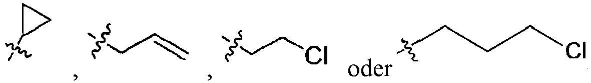 ist; und R₄ Wasserstoff oder Fluor ist; oder
(b) eine Verbindung mit der chemischen Struktur (II): 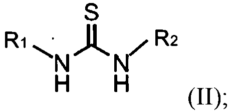
wobei R₁ 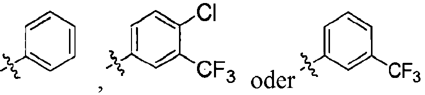 ist; und 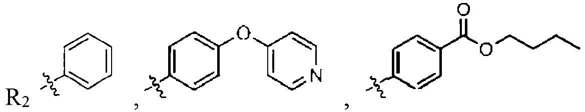 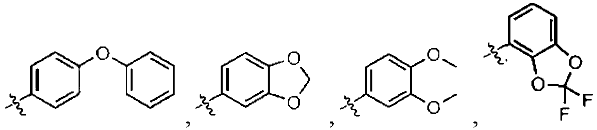 oder 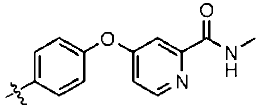 ist.

2. Die antimikrobielle Zusammensetzung zur Verwendung bei der Behandlung bakterieller Infektionen nach Anspruch 1, wobei die Phenylharnstoffverbindung gemäß Fall (a) ist und R₁ ist 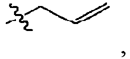 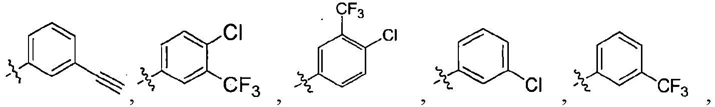 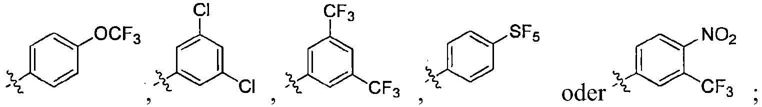 und R₂ ist

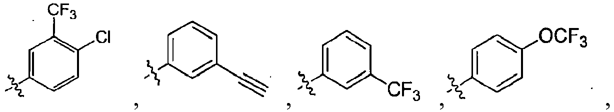

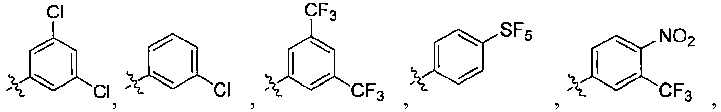

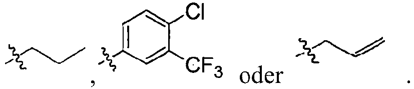

3. Die antimikrobielle Zusammensetzung zur Verwendung bei der Behandlung bakterieller Infektionen nach Anspruch 1, wobei die Phenylharnstoffverbindung eine Verbindung mit einer der chemischen Strukturen (III) bis (VII) ist:

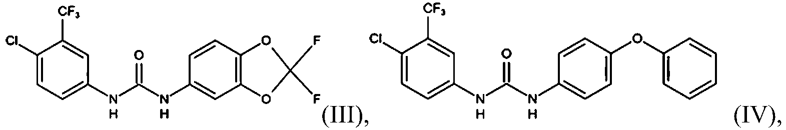

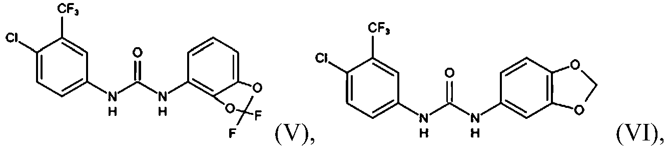

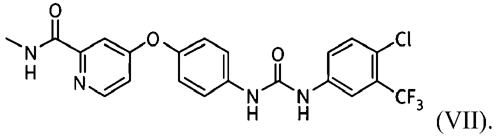

4. Die antimikrobielle Zusammensetzung zur Verwendung bei der Behandlung bakterieller Infektionen nach Anspruch 1, wobei die Fettsäure eine cis-mono-ungesättigte Fettsäure oder eine cis-poly-ungesättigte Fettsäure mit 16 bis 22 Kohlenstoffatome ist.

5. Die antimikrobielle Zusammensetzung zur Verwendung bei der Behandlung bakterieller Infektionen nach Anspruch 1, wobei die Fettsäure Palmitoleinsäure, Ölsäure, Linolsäure, α-Linolensäure, Arachidonsäure, Eicosapentaensäure oder Docosahexaensäure ist.

6. Die antimikrobielle Zusammensetzung zur Verwendung bei der Behandlung bakterieller Infektionen nach Anspruch 1, wobei der Gehalt an Fettsäure größer ist als der Gehalt an Phenylharnstoffverbindung.

7. Die antimikrobielle Zusammensetzung zur Verwendung bei der Behandlung bakterieller Infektionen nach einem der vorstehenden A, wobei die mikrobielle Zelle eine Zelle eines humanpathogenen Bakteriums ist.

8. Die antimikrobielle Zusammensetzung zur Verwendung bei der Behandlung bakterieller Infektionen nach Anspruch 7, wobei das humanpathogene Bakterium aus einer Gruppe ausgewählt ist, die aus *Staphylococcus aureus (S. aureus), Staphylococcus haemolyticus (S. haemolyticus), Staphylococcus hominis (S. hominis), Staphylococcus intermedius (S. intermedius), Staphylococcus saprophyticus (S. saprophyticus), Staphylococcus lugdunesis (S. lugdunesis), Erysipelothrix rhusiopathiae, Enterococcus faecalis*, *Enterococcus faecium, VR-E. faecium, Bacillus cereus, Bacillus subtilis, Corynebacterium diphtheriae, Listeria monocytogenes, Streptococcus pyogenes, Clostridium difficile, Escherichia coli, Salmonelia Typhimurium, Acinetobacter baumannii, Mycobacterium tuberculosis,* MRSA und Vancomycin-resistenter *Staphylococcus aureus* (VRSA).

9. Die antimikrobielle Zusammensetzung zur Verwendung bei der Behandlung bakterieller Infektionen gemäß einem der Ansprüche 1 bis 6, wobei der mikrobieller Biofilm ein Biofilm eines humanpathogenen Bakteriums oder Pilzes ist.

10. Die antimikrobielle Zusammensetzung zur Verwendung bei der Behandlung bakterieller Infektionen nach Anspruch 9, wobei das humanpathogene Bakterium oder der humanpathogene Pilz aus einer Gruppe ausgewählt ist, die aus *Staphylococcus haemolyticus, Klebsiella pneumoniae, Pseudomonas aeruginosa, Escherichia coli, Enterococcus faecalis*, *Enterococcus faecium, Candida albicans* und *Staphylococcus aureus* besteht.

11. Pharmazeutische Zusammensetzung, umfassend die antimikrobielle Zusammensetzung nach einem der Ansprüche 1 bis 10 und mindestens einen pharmazeutisch verträglichen Träger.

## Revendications

1. Une composition antimicrobienne destinée à être utilisée dans le traitement d'infections bactériennes pour inhiber la croissance d'une cellule microbienne et la croissance d'un biofilm microbien, ladite composition antimicrobienne comprenant :
un composé de phénylurée ; et
un acide gras ;
dans laquelle l'acide gras est un acide gras cis-insaturé ayant 16 à 22 atomes de carbone ; et
le composé de phénylurée est :
(a) un composé ayant la structure chimique (I-1) ou (I-2) : 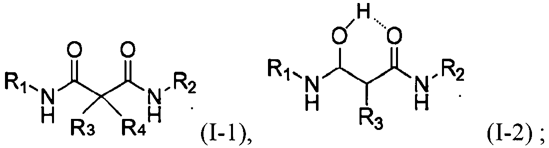 où chacun de R₁ et R₂ 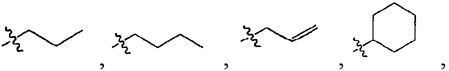 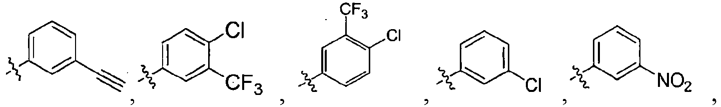 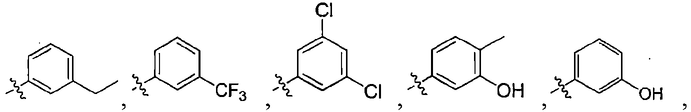 ou 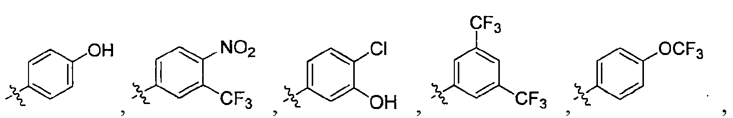 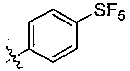 et R₁ et R₂ ne sont pas simultanément 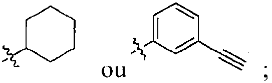 R₃ est un atome d'hydrogène, un groupe toluène, 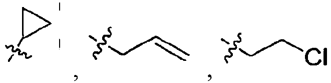 ou 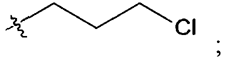 et R₄ est un atome d'hydrogène ou de fluor ; ou
(b) un composé ayant la structure chimique (II) : 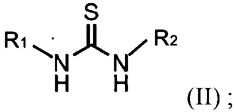 où R₁ est 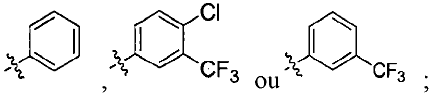 et R₂ représente 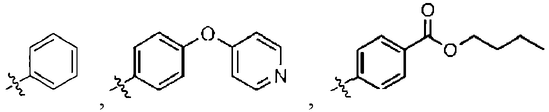 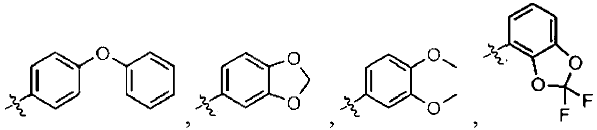 ou

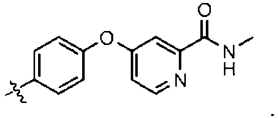

2. La composition antimicrobienne destinée à être utilisée dans le traitement d'infections bactériennes selon la revendication 1, dans laquelle le composé de phénylurée est celui du cas (a) et R_{(1) est} 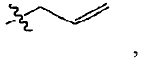 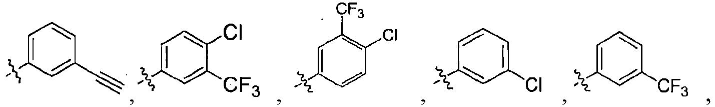 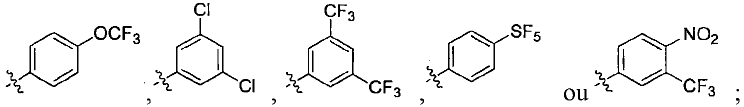 et R₂ est

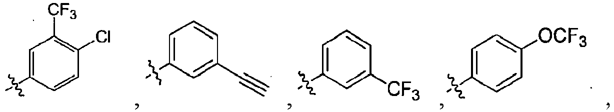

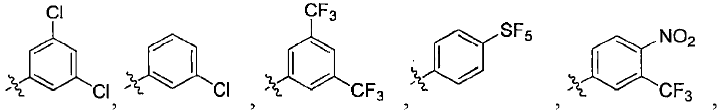

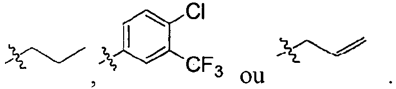

3. La composition antimicrobienne destinée à être utilisée dans le traitement d'infections bactériennes selon la revendication 1, dans laquelle le composé de phénylurée est un composé ayant l'une des structures chimiques (III) à (VII) :

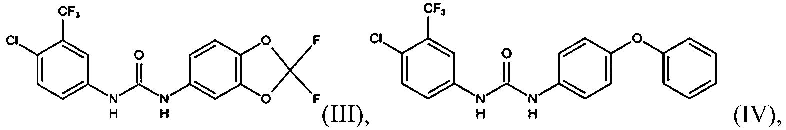

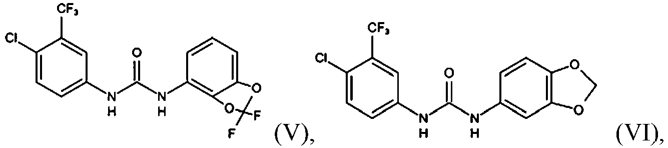

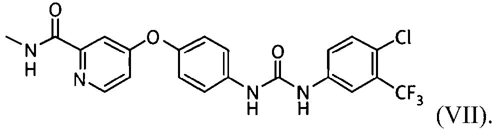

4. La composition antimicrobienne destinée à être utilisée dans le traitement d'infections bactériennes selon la revendication 1, dans laquelle l'acide gras est un acide gras cis-mono-insaturé ou un acide gras cis-poly-insaturé comportant 16 à 22 atomes de carbone.

5. La composition antimicrobienne destinée à être utilisée dans le traitement d'infections bactériennes selon la revendication 1, dans laquelle l'acide gras est l'acide palmitoléique, l'acide oléique, l'acide linoléique, l'acide α-linolénique, l'acide arachidonique, l'acide eicosapentaénoïque ou l'acide docosahexaénoïque.

6. La composition antimicrobienne destinée à être utilisée dans le traitement d'infections bactériennes selon la revendication 1, dans laquelle la teneur en acide gras est supérieure à la teneur en composé de phénylurée.

7. La composition antimicrobienne destinée à être utilisée dans le traitement d'infections bactériennes selon l'une des a s précédentes, dans laquelle la cellule microbienne est une cellule d'une bactérie pathogène pour l'homme.

8. La composition antimicrobienne destinée à être utilisée dans le traitement d'infections bactériennes selon la revendication 7, dans laquelle la bactérie pathogène pour l'homme est choisie dans un groupe comprenant *Staphylococcus aureus (S. aureus), Staphylococcus haemolyticus (S. haemolyticus), Staphylococcus hominis (S. hominis), Staphylococcus intermedius (S. intermedius), Staphylococcus saprophyticus (S. saprophyticus), Staphylococcus lugdunesis (S. lugdunesis), Erysipelothrix rhusiopathiae, Enterococcus faecalis*, *Enterococcus faecium, VR-E. faecium, Bacillus cereus, Bacillus subtilis, Corynebacterium diphtheriae, Listeria monocytogenes, Streptococcus pyogenes, Clostridium difficile, Escherichia coli, Salmonelia Typhimurium, Acinetobacter baumannii, Mycobacterium tuberculosis,* MRSA et *Staphylococcus aureus* résistant à la vancomycine (VRSA).

9. La composition antimicrobienne destinée à être utilisée dans le traitement d'infections bactériennes selon l'une des revendications 1 à 6, dans laquelle le biofilm microbien est un biofilm d'une bactérie ou d'un champignon pathogène pour l'homme.

10. La composition antimicrobienne destinée à être utilisée dans le traitement d'infections bactériennes selon la revendication 9, dans laquelle la bactérie ou le champignon pathogène pour l'homme est choisi dans un groupe constitué de *Staphylococcus haemolyticus, Klebsiella pneumoniae, Pseudomonas aeruginosa, Escherichia coli, Enterococcus faecalis, Enterococcus faecium, Candida albicans* et *Staphylococcus aureus.*

11. Composition pharmaceutique comprenant la composition antimicrobienne selon l'une des revendications 1 à 10 et au moins un excipient pharmaceutiquement acceptable.
